# FASCICULE DE BREVET EUROPEEN

(11) **EP 3 749 949 B1**
(45) Date de publication et mention de la délivrance du brevet: **13.09.2023**
(21) Numéro de dépôt: 19710449.0
(22) Date de dépôt: 05.02.2019
(51) Int. Cl.: G01N 21/3563, G01N 21/3581, G01N 21/3586

(54) **SYSTÈME D'IMAGERIE TÉRAHERTZ À RÉFLEXION**
TERAHERTZ-REFLEXIONSBILDGEBUNGSSYSTEM
TERAHERTZ REFLECTION IMAGING SYSTEM

(30) Priorité: 07.02.2018 FR 1851025
(43) Date de publication de la demande: 16.12.2020
(73) Titulaire: Tihive, 38240 Meylan (FR)
(72) Inventeur: SHERRY, Hani, 38000 Grenoble (FR)
(74) Mandataire: de Jong, Jean Jacques
(86) Numéro de dépôt international: PCT/FR2019/050254
(87) Numéro de publication internationale: WO 2019/155156

(56) Documents cités:
- WO-A1-2016/185010
- WO-A2-2016/196309
- US-A1- 2001 029 436
- US-A1- 2002 005 951
- US-A1- 2014 326 890
- US-A1- 2014 367 575
- SENGUPTA KAUSHIK ET AL: "Silicon Integrated 280 GHz Imaging Chipset With 4$\times$ 4 SiGe Receiver Array and CMOS Source", IEEE TRANSACTIONS ON TERAHERTZ SCIENCE AND TECHNOLOGY, IEEE, PISCATAWAY, NJ, USA, vol. 5, no. 3, 1 mai 2015 (2015-05-01), pages 427-437, XP011579783, ISSN: 2156-342X, DOI: 10.1109/TTHZ.2015.2414826 [extrait le 2015-04-29]
- PFEIFFER ULLRICH R ET AL: "A 0.53 THz Reconfigurable Source Module With Up to 1 mW Radiated Power for Diffuse Illumination in Terahertz Imaging Applications", IEEE JOURNAL OF SOLID-STATE CIRCUITS, IEEE SERVICE CENTER, PISCATAWAY, NJ, USA, vol. 49, no. 12, 1 décembre 2014 (2014-12-01), pages 2938-2950, XP011564903, ISSN: 0018-9200, DOI: 10.1109/JSSC.2014.2358570 [extrait le 2014-11-20]
- PFEIFFER ULLRICH R ET AL: "Silicon-based sources and detectors for terahertz applications", 2014 CONFERENCE ON LASERS AND ELECTRO-OPTICS (CLEO) - LASER SCIENCE TO PHOTONIC APPLICATIONS, THE OPTICAL SOCIETY, 8 juin 2014 (2014-06-08), pages 1-2, XP032707628, DOI: 10.1364/CLEO_SI.2014.STU1F.3
- ANONYMOUS: "Millimeter-wave imaging using silicon technology", VLSI DESIGN, AUTOMATION AND TEST (VLSI-DAT), 2011 INTERNATIONAL SYMPOSIUM ON, IEEE, 25 avril 2011 (2011-04-25), pages 1-21, XP031880500, DOI: 10.1109/VDAT.2011.5783567 ISBN: 978-1-4244-8500-0
- JIANG CHEN ET AL: "25.5 A 320GHz subharmonic-mixing coherent imager in 0.13 m SiGe BiCMOS", 2016 IEEE INTERNATIONAL SOLID-STATE CIRCUITS CONFERENCE (ISSCC), IEEE, 31 janvier 2016 (2016-01-31), pages 432-434, XP032873713, DOI: 10.1109/ISSCC.2016.7418092 ISBN: 978-1-4673-9466-6 [extrait le 2016-02-23]
- IWAMI K ET AL: "Design and Fabrication of a Scanning Near-Field Microscopy Probe with Integrated Zinc Oxide Photoconductive Antennas for Local Terahertz Spectroscopy", SENSORS AND MATERI, SCIENTIFIC PUBLISHING DIVISION OF MYU, TOKYO, JP, vol. 22, no. 3, 1 janvier 2010 (2010-01-01), pages 135-142, XP008140640, ISSN: 0914-4935

## Description

### Domaine technique

L'invention concerne les techniques d'imagerie à réflexion, utilisant notamment des sondes à placer à proximité d'un objet à analyser. L'invention explore dans ce contexte des applications des ondes du domaine térahertz.

### Arrière-plan

Le domaine des ondes térahertz (THz) est compris entre les ondes millimétriques et le rayonnement visible. On admet que le domaine térahertz s'étend en fréquence d'environ 300 GHz à quelques THz. Dans ce domaine, les ondes ont des propriétés à la fois du domaine radiofréquence et du domaine optique - elles peuvent en particulier être émises et reçues par des antennes, et être focalisées par des systèmes optiques, par exemple des lentilles en silicium.

Les ondes THz ont la propriété de traverser certains objets sans avoir la nocivité des rayons-X. En imagerie médicale, on les utilise, par exemple, pour détecter des tissus cancéreux, du fait que ces tissus ont des propriétés d'absorption et réflexion différentes des tissus sains dans le domaine THz.

L'article ["Use of a handheld terahertz pulsed imaging device to differentiate benign and malignant breast tissue", Maarten R. Grootendorst et al., Vol. 8, No. 6, 1 Jun 2017, Biomédical Optics Express 2932] décrit une sonde portative conçue pour être glissée au contact de la peau d'un patient et analyser celle-ci par réflexion d'ondes, à la façon d'une sonde d'échographie.

Les ondes THz sont mises en oeuvre dans la sonde par l'intermédiaire d'impulsions laser femtoseconde générées hors de la sonde et guidées le long de fibres optiques vers un émetteur/récepteur photoconducteur placé dans la sonde. Des impulsions résultantes de 0,1 à 1,8 THz sont ensuite guidées par un miroir oscillant entre l'émetteur/récepteur et une fenêtre de quartz présente à l'extrémité de la sonde, pour balayer pas à pas 26 pixels dans une zone de 15 × 2 mm à une fréquence de 4 Hz. A chaque pas du balayage, des impulsions THz réfléchies sont renvoyées par le pixel correspondant vers le récepteur.

Une telle sonde portative met en oeuvre des technologies optiques complexes et coûteuses. En outre, le pas des pixels, de l'ordre de 0,6 mm, offre une résolution relativement basse. Cette résolution est contrainte par la précision du mécanisme entraînant le miroir et la longueur d'onde relativement importante des ondes THz. Le pas de 0,6 mm des pixels correspond sensiblement à la limite de diffraction d'Abbe dans l'air pour la fréquence la plus basse des impulsions utilisées, ici 0,1 THz et une longueur d'onde de 1,2 mm.

Un tel système requiert ainsi un équipement encombrant et coûteux pour mettre en oeuvre un capteur d'image de seulement 15 × 2 mm, l'essentiel de l'encombrement étant constitué par l'équipement de mise en oeuvre des faisceaux laser nécessaires.

On est récemment parvenu à réaliser des récepteurs et des émetteurs THz à l'aide de technologies des semi-conducteurs, qui sont entièrement exploitables par des circuits électroniques intégrés sur les mêmes puces.

On trouve ainsi des récepteurs THz regroupés dans une matrice sur une puce en semi-conducteur pour former un capteur d'image compact. Par exemple, l'article ["A 1 k-Pixel Video Camera for 0.7-1.1 Terahertz Imaging Applications in 65-nm CMOS", Richard Al Hadi, Hani Sherry, et al., IEEE Journal of Solid-State Circuits, VOL. 47, NO. 12, December 2012] décrit un capteur d'image formé de récepteurs THz entièrement réalisés en technologie CMOS 65 nm. Les récepteurs parviennent à traiter des signaux de fréquence supérieure à la fréquence de fonctionnement des transistors grâce à l'utilisation d'éléments passifs et de configurations où les transistors sont moins limités en fréquence (transistors montés en source commune). En particulier, on utilise une configuration de détection de puissance - les ondes THz sont reçues sur une antenne et le signal d'antenne résultant est redressé pour charger un condensateur à la valeur crête des alternances du signal. De tels récepteurs, dits homodynes, ne fournissent pas d'information de phase, mais seulement une information d'amplitude.

On est également parvenu à concevoir des émetteurs THz intégrables en technologie des semi-conducteurs, notamment CMOS. Une difficulté pour les émetteurs était de produire des signaux THz de fréquence supérieure à la fréquence de fonctionnement des transistors. Cette difficulté a été levée en utilisant des oscillateurs dits harmoniques. Un tel oscillateur fonctionne à une fréquence compatible avec la technologie et produit des harmoniques exploitables dans le domaine THz. Le brevet US9083324 décrit un tel oscillateur.

De plus amples informations sur des récepteurs et émetteurs THz intégrables peuvent être obtenues dans les thèses de Hani Sherry et Richard Al Hadi soutenues à l'université de Wuppertal en 2013.

Malgré la faisabilité démontrée d'une intégration de composants THz sur des puces de semi-conducteur, on n'est pas parvenu à proposer des sondes à réflexion compactes pouvant remplacer celle décrite dans l'article susmentionné de Biomédical Optics Express.

US 2014/326890 A1 divulgue un capteur pour système d'imagerie térahertz intégralement réalisable dans une technologie de semi-conducteurs comprenant une zone d'analyse, un substrat plan en matériau semi-conducteur transparent aux rayonnements térahertz, une pluralité de récepteurs d'un rayonnement térahertz agencés selon une matrice dans le substrat, une source de rayonnement térahertz agencée dans le substrat, le rayonnement étant réfléchi par la zone d'analyse sur la matrice de récepteurs.

### Résumé

L'invention est exposée dans le jeu de revendications annexé selon lequel la revendication indépendante 1 vise à un capteur pour système d'imagerie térahertz, intégralement réalisable dans une technologie de semi-conducteurs et la revendication indépendante 9 vise à un procédé d'imagerie térahertz à réflexion sans lentille utilisant un capteur.

Le pas de la matrice peut être au moins égal à la moitié de la longueur d'onde du rayonnement à l'intérieur du substrat, et l'émetteur prend la place d'un récepteur dans la matrice.

Les récepteurs peuvent être des récepteurs hétérodynes synchronisés sur un même oscillateur local, d'où il résulte que chaque récepteur fournit une information de phase représentative de la distance parcourue depuis l'émetteur par le rayonnement reçu.

Les récepteurs et l'émetteur peuvent avoir une configuration hexagonale et être agencés selon une matrice en nid d'abeille.

Le capteur peut comprendre plusieurs émetteurs régulièrement espacés dans la matrice.

Le capteur peut comprendre un support tubulaire configuré pour être appuyé sur l'objet et maintenir le capteur à une distance fixe de l'objet.

Le substrat peut avoir une épaisseur au plus égale à la moitié de la longueur d'onde du rayonnement térahertz à l'intérieur du substrat, et comprendre une face active incluant des niveaux de métal ; et une face arrière configurée pour être orientée vers l'objet à analyser. Chaque récepteur et émetteur peut alors comprendre une antenne annulaire formée dans un niveau de métal de la face active, la circonférence moyenne de l'antenne étant au moins égale à la moitié de la longueur d'onde du rayonnement térahertz à l'intérieur du substrat ; et un anneau de garde entourant l'antenne à la périphérie du récepteur ou émetteur, formé à partir de plages métalliques empilées sur plusieurs niveaux de métal.

L'anneau de garde peut comprendre des plages métalliques structurées pour former une cavité abritant des pistes conductrices et des composants électroniques servant à exploiter les récepteurs et l'émetteur.

Un procédé d'imagerie térahertz à réflexion sans lentille peut exploiter un capteur du type susmentionné à un seul émetteur, selon des étapes consistant à activer l'émetteur ; mesurer les signaux produits par les récepteurs en réponse à l'activation de l'émetteur pour former une image dans le domaine fréquentiel ; et opérer une transformée de Fourier inverse sur l'image du domaine fréquentiel pour produire une image dans le domaine spatial.

Un procédé d'imagerie térahertz à réflexion sans lentille peut exploiter un capteur du type susmentionné à plusieurs émetteurs, selon des étapes consistant à activer les émetteurs un par un selon une séquence résultant en l'illumination d'une même zone de l'objet à analyser selon des angles différents ; mesurer les signaux produits par les récepteurs en réponse à chaque activation dans la séquence pour former une image respective dans le domaine fréquentiel ; et traiter les multiples images du domaine fréquentiel pour produire une image améliorée dans le domaine spatial.

Les multiples images peuvent être traitées par une technique de ptychographie utilisant seulement des informations d'amplitude fournies par les récepteurs.

Les multiples images peuvent également être traitées par une technique de microscopie à synthèse d'ouverture utilisant des informations d'amplitude et de phase fournies par les récepteurs.

### Description sommaire des dessins

Des modes de réalisation seront exposés dans la description suivante, faite à titre non limitatif en relation avec les figures jointes parmi lesquelles :
- les figures 1A à 1C représentent, selon des vues de côté schématiques, un mode de réalisation d'imageur THz à réflexion en différentes étapes de l'acquisition d'une zone plane d'un objet à analyser ;
- la figure 2A représente une vue de face schématique de l'imageur des figures 1A à 1C, révélant un exemple d'agencement des émetteurs ;
- la figure 2B illustre une collection de zones illuminées par l'imageur de la figure 2A ;
- la figure 3 représente une vue de face schématique d'un autre mode de réalisation d'imageur THz à réflexion, formé de pixels émetteurs et récepteurs hexagonaux agencés dans une matrice en nid d'abeille ;
- la figure 4 représente une vue de face schématique d'une variante d'imageur en nid d'abeille ;
- la figure 5 représente une vue de dessus agrandie d'un mode de réalisation de pixels hexagonaux réalisés dans une technologie des semi-conducteurs ; et
- la figure 6 représente une vue en coupe d'un exemple de configuration des pixels de la figure 6.

### Description de modes de réalisation

Jusqu'à présent, les émetteurs et récepteurs THz sur puce ont été envisagés pour des usages en dispositifs dissociés, notamment agencés de part et d'autre d'un objet à analyser par transmission. Dans cette configuration, chaque dispositif est muni d'une lentille en silicium coûteuse et relativement encombrante par rapport aux dimensions des puces.

Si on a proposé dans l'état de l'art d'intégrer une matrice de plusieurs émetteurs sur une puce, c'était pour pouvoir adapter l'intensité d'émission (et donc la consommation) aux conditions de mesure en activant plus ou moins d'émetteurs à la fois (thèse de Richard Al Hadi).

On n'a pas envisagé sérieusement dans l'état de l'art d'utiliser des récepteurs THz sur puce en mode réflexion, car les récepteurs les plus répandus, des détecteurs de puissance, ne fournissent pas d'informations de phase, ce qui poserait des problèmes liés à la réflexion spéculaire de l'objet analysé, comme l'indique l'article IEEE susmentionné.

Malgré cela, on propose ici d'utiliser une matrice de récepteurs sur puce en mode réflexion, et d'intégrer sur la même puce des émetteurs parmi les récepteurs. Une telle configuration semble a priori contraire à des règles préconçues. En effet, dans les domaines radiofréquence et optique voisins, il n'est pas concevable de placer des émetteurs à proximité immédiate des récepteurs car les émetteurs perturberaient les récepteurs voisins (couplages ou réflexions parasites).

Il s'avère que les ondes THz sont relativement faciles à maîtriser dans une puce de semi-conducteur, et que les problèmes de perturbation peuvent être contenus par des moyens simples exposés ci-après. Il en résulte que la juxtaposition d'un émetteur et d'un récepteur sur une puce ne pose aucun problème en prenant des précautions élémentaires. De plus, les émetteurs et les récepteurs peuvent être réalisés aux mêmes dimensions, de sorte qu'ils peuvent être mélangés de façon quelconque dans une matrice sans en perturber le pas.

La réflexion spéculaire dans le domaine THz est en fait un faux problème, même en utilisant des détecteurs de puissance (qui ne fournissent pas d'information de phase permettant d'évaluer la distance). En effet, dans certaines applications, la seule information de la quantité de rayonnement réfléchi peut être importante, car on peut avec les ondes THz différencier des zones par leur réflectivité, ce qui correspond par exemple aux applications de détection de tissus cancéreux.

En outre, on envisage ici d'utiliser l'imageur résultant sans lentille, car il s'avère qu'on peut alors appliquer de façon commode des techniques d'imagerie sans lentille utilisées dans d'autres domaines, comme on l'exposera ci-après. L'imageur convient alors particulièrement bien à l'analyse d'un objet proche par réflexion, par exemple à une distance de l'ordre du centimètre, ce qui facilite la conception des émetteurs compte tenu de la faible puissance d'émission alors requise.

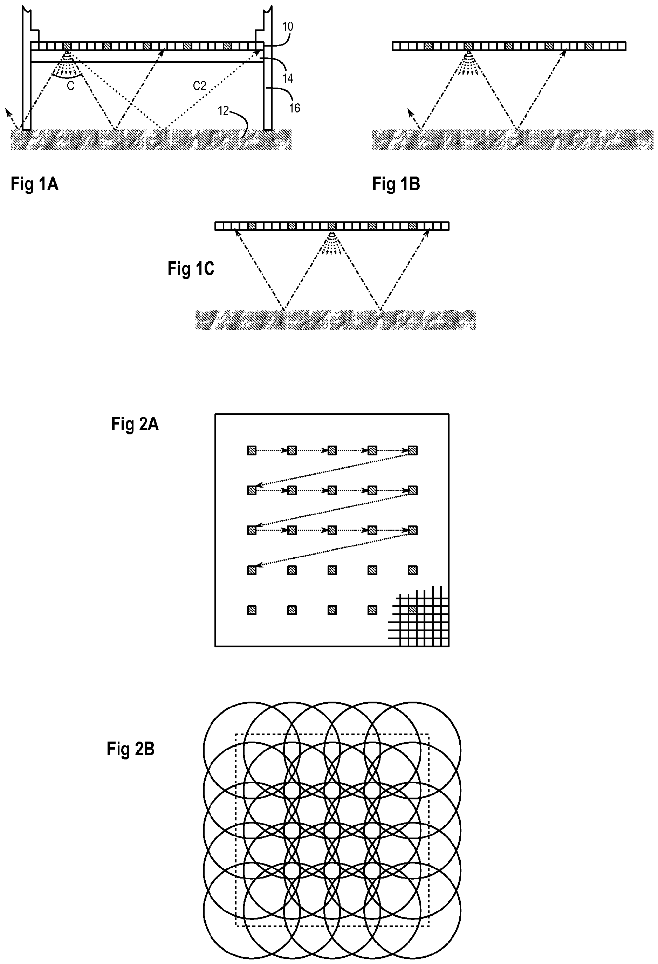

Les figures 1A à 1C représentent, selon des vues de côté schématiques, un mode de réalisation d'imageur THz à réflexion 10 en différentes étapes de l'acquisition d'une zone plane d'un objet à analyser 12.

Comme le montre en particulier la figure 1A, l'imageur 10 est formé sur un substrat plat en matériau semi-conducteur comprenant une pluralité de pixels agencés en une matrice. Une rangée de pixels de la matrice est illustrée par des carrés. Des carrés blancs représentent des récepteurs, et des carrés gris représentent des émetteurs. A titre d'exemple, un pixel sur cinq est un émetteur.

La matrice de pixels 10 est formée sur une face dite active du substrat, face qui est tournée vers le haut, à l'opposé de l'objet 12. La partie plus épaisse du substrat 14, dite face arrière, est tournée vers l'objet 12. En effet, les récepteurs THz présentent une meilleure sensibilité, et les émetteurs produisent un signal plus fort, du côté plus épais du substrat.

L'imageur peut être maintenu à une distance fixe de l'objet 12 par un support tubulaire 16, en un matériau qui, de préférence, ne réfléchit pas les ondes THz. On réalise ainsi une sonde de type stylo que l'on peut appliquer sur des zones à analyser.

Dans un mode de réalisation, on prévoit d'activer les pixels émetteurs un par un dans une séquence - les figures 1A à 1C illustrent les activations des trois premiers émetteurs de la rangée en partant de la gauche.

A la figure 1A, le premier pixel émetteur de la rangée est activé, le cinquième à partir du bord gauche. Il émet des ondes THz selon un cône d'émission C qui, selon la structure de l'émetteur, peut atteindre un angle d'ouverture de 90° (on a représenté à titre d'exemple un angle d'ouverture d'environ 60°). Le cône forme sur la surface de l'objet 12 un disque qui est réfléchi vers l'imageur dans la continuité d'un cône symétrique. L'imageur reçoit un disque de diamètre deux fois supérieur à celui reçu par l'objet, normalement centré sur l'émetteur.

L'émetteur activé se comportant comme une source ponctuelle cohérente, le disque reçu par l'imageur est une transformée de Fourier spatiale, ou hologramme, de la zone éclairée sur l'objet 12. Cet hologramme comporte des informations d'amplitude et de phase permettant, par transformée de Fourier inverse, de reconstituer une représentation spatiale tridimensionnelle de l'objet tel qu'aperçu depuis l'émetteur activé.

Selon la nature des matériaux présents dans l'objet, les ondes THz peuvent être transmises, absorbées ou réfléchies avec des degrés variables. Ainsi les ondes peuvent traverser une surface transmissive de l'objet et être réfléchies par des matières présentes plus en profondeur. L'objet peut être la peau d'un patient que l'on investigue pour détecter des tissus cancéreux - les tissus cancéreux ont un taux de réflexion plus élevé que les tissus sains, ce qui permet de les différencier par une analyse de la réflexion.

Si on parvient à exploiter les informations de phase de l'hologramme à l'aide des récepteurs, on pourra reconstruire une représentation tridimensionnelle des tissus réfléchissants. On pourra alternativement n'exploiter que les informations de phase, qui peuvent dans certaines applications révéler des propriétés particulières de l'objet analysé.

Si on parvient seulement à exploiter les informations d'amplitude de l'hologramme, par exemple si les récepteurs sont des détecteurs de puissance, on obtient quand même une projection des niveaux de réflexion, permettant de déceler des zones plus ou moins réfléchissantes, et ainsi détecter les contours de tissus cancéreux.

Dans la configuration représentée, avec l'angle d'ouverture de 60°, le disque reçu dans le plan de l'imageur ne couvre pas l'ensemble de l'imageur, signifiant que les pixels de l'imageur sont sous-exploités. Pour améliorer la précision de l'hologramme, on souhaite exploiter tous les pixels de l'imageur. Pour cela, on peut augmenter l'angle d'ouverture du cône, comme cela est représenté par une ligne C2, de sorte que le pixel le plus éloigné de l'imageur reçoive un rayon réfléchi du bord du disque éclairé de l'objet.

Selon une alternative, on peut éloigner l'imageur de l'objet, ce qui a pour résultat d'agrandir les disques proportionnellement à la distance.

Avec un seul émetteur, placé au centre de l'imageur, on pourra au plus observer un disque de la moitié du diamètre de l'imageur. Pour couvrir toute la zone de l'objet en face de l'imageur, on peut avoir recours à plusieurs émetteurs répartis dans l'imageur, comme cela est représenté. Par contre, on ne prévoit pas d'activer plusieurs émetteurs en même temps, car cela provoquerait des interférences complexes à traiter dans l'image capturée par l'imageur.

Ainsi, comme le montrent les figures 1A à 1C pour les trois premiers émetteurs de la rangée, on envisage d'activer les émetteurs un par un en séquence, et d'enregistrer séparément les images résultantes capturées par l'imageur.

La figure 2A illustre, à l'échelle de la figure 1A, l'extension de cette séquence d'activation pour un exemple d'imageur vu de face, où les émetteurs sont distribués avec un taux de un sur cinq par rangées et par colonnes.

La figure 2B illustre, à l'échelle de la figure 2A, la collection de disques observés sur l'objet 12 comme suite à l'activation de tous les émetteurs de la figure 2A. Un carré en pointillés illustre la zone effectivement observée compte tenu des bords de l'imageur.

Il s'avère que la collection d'images résultantes capturées par l'imageur, dans le domaine de Fourier, peut être traitée selon des techniques connues dans d'autres domaines pour reconstituer une image de toute la zone éclairée, voire améliorer le rendu de l'objet.

Les seules informations d'amplitude des images capturées selon l'exemple qui vient d'être décrit peuvent être traitées par la technique dite ptychographie, utilisée dans l'imagerie à rayons-X pour récupérer des informations de phase. On peut ainsi reconstruire une représentation spatiale de l'objet observé (ici par réflexion au lieu de transmission pour les rayons-X).

Les zones de l'objet qui se trouvent à l'intersection du plus grand nombre de cônes d'émission permettent d'atteindre un meilleur rendu tridimensionnel, car ces zones sont éclairées selon le plus grand nombre d'angles. Dans l'exemple de la figure 2B, ces zones sont celles en face des neuf pixels émetteurs centraux de la matrice.

Ainsi, pour améliorer le rendu tridimensionnel, on peut agrandir les angles d'émission ou éloigner l'imageur de l'objet. Cela permet d'étendre les zones d'intersection des cônes d'émission et tendre vers une configuration optimale où une zone d'intérêt centrale de l'objet se situe à l'intersection de tous les cônes. Il convient bien entendu de tenir compte de l'angle de réception des récepteurs - dans le domaine THz, les récepteurs et les émetteurs ont des structures similaires menant à des angles d'émission et de réception proches, atteignant 90°.

On peut également augmenter le nombre de pixels émetteurs pour améliorer le rendu tridimensionnel. Cependant, chaque pixel émetteur remplace un pixel récepteur dans la matrice, de sorte que l'image capturée par l'imageur comporte un pixel « noir » à cet endroit. Les valeurs de tels pixels noirs peuvent être interpolées à partir des pixels récepteurs adjacents. On peut trouver un compromis entre le gain obtenu par l'augmentation du nombre d'émetteurs et la perte de détail restitué par l'imageur du fait de la diminution de pixels récepteurs.

Dans la configuration représentée à la figure 1A, l'imageur est à une distance connue de l'objet. On peut ainsi calculer l'angle d'incidence du rayonnement parvenant à chaque récepteur en fonction des coordonnées du récepteur et de l'émetteur activé. Cela permet, en fonction des lobes de sensibilité et de puissance connus du récepteur et de l'émetteur, de corriger l'intensité mesurée, et d'augmenter l'angle utile des cônes d'émission et de réception.

Si les récepteurs permettent de mesurer la phase, on peut utiliser une technique dite de microscopie à synthèse d'ouverture ou SAM (Synthetic Aperture Microscopy). Une telle technique est décrite, par exemple, dans l'article ["Theory of the Synthetic Aperture Microscope", Terry Turpin et al., SPIE Proceedings, Vol. 2566B-34, July 1995, San Diego]. Cette technique vise à reconstruire une représentation tridimensionnelle précise de l'objet observé à partir de vues selon différents angles et des informations de profondeur (phase) obtenues dans chaque vue. Comme les informations de phase ne sont pas limitées par la longueur d'onde ni le pas des pixels, on parvient à reconstruire une représentation tridimensionnelle dont la résolution tend vers la précision de la mesure de phase. A partir de la représentation tridimensionnelle on peut alors extraire des coupes dont la résolution dépasse la limite de diffraction d'Abbe ou le pas des pixels.

On remarquera que dans le domaine THz, on parvient à mesurer la phase directement à l'aide de circuits électroniques, alors que dans le domaine optique des microscopes, la phase est mesurée par interférence de deux faisceaux cohérents. Ainsi, dans l'article de SPIE Proceedings susmentionné, on propose un système optique complexe pour faire interférer un faisceau laser principal avec un faisceau laser réfléchi sur l'objet à analyser, émis par une source cohérente qui, grâce à un système mécanique, éclaire l'objet selon une multitude d'angles en séquence.

Ainsi, l'utilisation de la technique SAM implique l'utilisation de récepteurs THz capables de mesurer la phase, par exemple des récepteurs hétérodynes. L'article ["A Fully Integrated 320 GHz Cohérent Imaging Transceiver in 130 nm SiGe BiCMOS", Chen Jiang et al., IEEE Journal of Solid-State Circuits, Vol. 51, No. 11, November 2016] ainsi que la thèse de Hani Sherry décrivent des mises en oeuvre de récepteurs THz hétérodynes en technologie des semi-conducteurs. Un récepteur hétérodyne mélange le signal provenant d'un oscillateur local fixe et le signal THz reçu pour produire un signal de fréquence intermédiaire (différence des fréquences) exploitable sur la puce, et qui véhicule l'information de phase. Le signal d'oscillateur local peut être produit de la même manière que le signal des émetteurs (par exemple un oscillateur harmonique), en choisissant une fréquence légèrement décalée pour que la fréquence intermédiaire ne soit pas nulle. On peut pour cela utiliser deux oscillateurs de même structure, mais alimentés à des tensions différentes pour obtenir le décalage. On peut également utiliser deux boucles à verrouillage de phase utilisant la même fréquence de référence, mais avec des facteurs de multiplication différents.

Le signal de fréquence intermédiaire a alors une amplitude proportionnelle à l'amplitude du signal THz reçu et une phase égale à la phase que l'on souhaite mesurer. Tous les récepteurs de l'imageur sont alors alimentés par le même oscillateur local de sorte qu'ils soient synchrones et aient la même référence pour mesurer la phase.

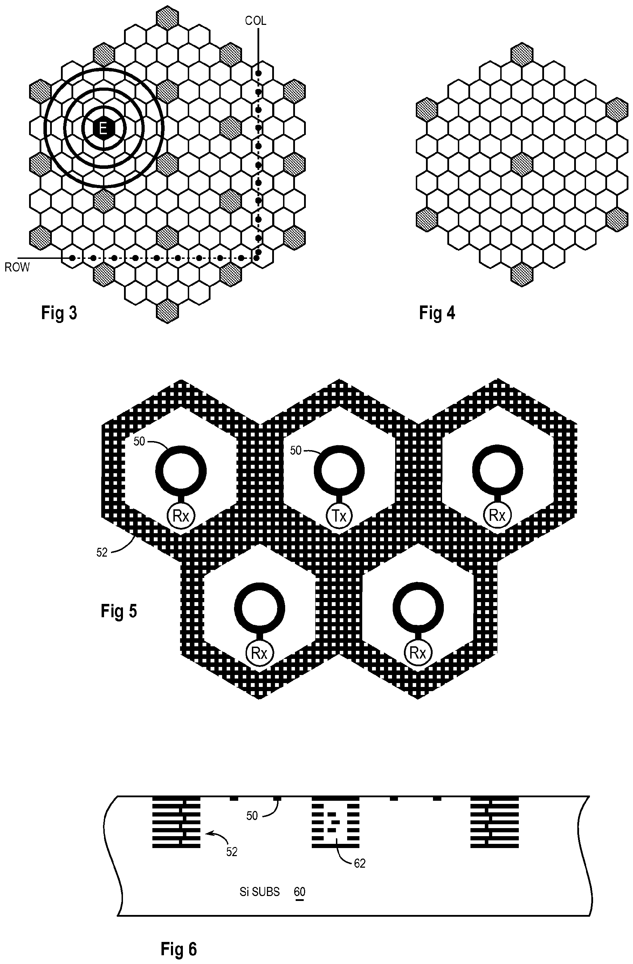

La figure 3 représente une vue de face schématique d'un autre mode de réalisation d'imageur THz à réflexion. L'imageur comprend des pixels émetteurs et récepteurs hexagonaux agencés selon une matrice en nid d'abeille. La matrice comporte des rangées de pixels juxtaposés selon trois axes principaux à 0° (horizontal), 120° et 240°. Selon chaque axe principal, un pixel sur six est un pixel émetteur. Les pixels émetteurs sont en outre répartis dans la matrice pour que chacun soit équidistant des six autres pixels émetteurs les plus proches, disposés aux sommets d'un hexagone.

La configuration en hexagone des pixels est particulièrement bien adaptée à la structure des émetteurs et récepteurs THz envisagés. En effet, ceux-ci peuvent être basés sur une antenne annulaire, comme on le verra ci-après, et la structure hexagonale est plus compacte qu'une structure carrée pour contenir une antenne annulaire. Du fait en outre que la matrice est en nid d'abeille, celle-ci peut loger un plus grand nombre de pixels pour un pas donné entre pixels. Ces caractéristiques combinées permettent d'obtenir une résolution sensiblement plus élevée pour un pas donné qu'une matrice carrée et un meilleur rendu de lignes obliques.

Par ailleurs, la structure en nid d'abeille s'avère offrir une meilleure précision dans l'application envisagée. Comme on l'a représenté pour un émetteur E, celui-ci émet selon un cône qui est réfléchi vers l'émetteur et les récepteurs qui l'entourent selon des anneaux concentriques, en supposant que la surface de l'objet est sensiblement plane. Les anneaux peuvent être considérés comme des anneaux d'égale intensité, la valeur de l'intensité allant en diminuant radialement à partir du centre, selon le lobe de puissance d'émission de l'émetteur. Chaque anneau peut être contenu, comme cela est représenté, dans des pixels juxtaposés formant un contour hexagonal qui approxime fidèlement l'anneau. Cela permet notamment d'obtenir une meilleure restitution de motifs concentriques caractéristiques d'une transformée de Fourier spatiale et d'appliquer de façon plus uniforme un facteur de correction de l'intensité en fonction de l'angle d'incidence.

La commande des pixels de la matrice en nid d'abeille ne pose de pas problème particulier par rapport à des pixels d'une matrice carrée. Comme on l'a représenté à titre d'exemple, les pixels de chaque rangée à 0° peuvent être commandés par des lignes de rangée ROW, et les pixels appartenant à chaque paire de diagonales adjacentes à 90° peuvent être commandés par des lignes de colonne COL. La gestion particulière de récepteurs et émetteurs THz en matrice est connue et ne sera pas décrite plus en détail ici.

La figure 4 représente un autre exemple de répartition d'émetteurs dans une matrice en nid d'abeille. Alors qu'à la figure 3 chaque émetteur était entouré de trois hexagones concentriques complets, chaque émetteur est ici entouré de cinq hexagones concentriques complets.

La figure 5 représente une vue de dessus partielle agrandie d'un mode de réalisation de pixels hexagonaux réalisés dans une technologie des semi-conducteurs, par exemple CMOS 65 nm. On a représenté un pixel émetteur Tx entouré de quatre pixels récepteurs adjacents Rx. Les éléments de cette vue sont sensiblement à l'échelle pour un imageur conçu pour travailler à environ 600 GHz, à titre d'exemple. Comme on le voit, tous les pixels, récepteurs et émetteurs, sont identiques quant aux dimensions.

La fréquence de 600 GHz correspond à une longueur d'onde de 0,5 mm dans l'air. Les pixels sont intégrés dans un substrat en silicium, où la longueur d'onde subit une réduction d'un facteur multiplicatif d'environ 0,6, ramenant la longueur d'onde à environ 0,3 mm dans le silicium. Par ailleurs, on accepte de ne travailler que sur la moitié de la longueur d'onde, soit 0,15 mm, car cela permet d'augmenter la résolution d'un facteur 2 avec une perte acceptable de gain. Ainsi, les antennes 50 des émetteurs et récepteurs sont dimensionnées pour travailler avec cette longueur d'onde. Les antennes 50 sont ici annulaires, ce qui implique que leur circonférence moyenne est au moins égale à la longueur d'onde de travail, soit 0,15 mm.

Les anneaux sont réalisés, par exemple, dans la dernière couche de métal de la technologie et ont une largeur de 10 µm, soit un diamètre externe de 64 µm et un diamètre interne de 54 µm.

Par ailleurs, pour éviter la propagation transversale de perturbations électriques par couplage inductif ou capacitif entre pixels, chaque pixel comprend un anneau de garde périphérique 52, anneau qui peut être circulaire ou, ici, hexagonal. L'antenne est centrée dans une zone majoritairement dépourvue de métal dont le diamètre moyen est sensiblement égal à la longueur d'onde de travail (0,15 mm). Ainsi, le bord intérieur de l'anneau de garde est au moins à 38 µm du bord externe de l'anneau d'antenne. L'anneau de garde a par ailleurs une largeur de 30 µm, et est structuré pour respecter un rapport métal/vide préconisé par la technologie. Le pixel a ainsi une largeur de 200 µm entre deux faces opposées de l'hexagone, valeur correspondant au pas selon chacun des trois axes à 0°, 120° et 240°.

La figure 6 est une vue en coupe des pixels de la figure 5. Les pixels sont formés sur la face active d'un substrat semi-conducteur 60, ici en silicium. Les antennes 50, réalisées dans le dernier niveau de métal, affleurent la face supérieure du substrat. Cette face supérieure est normalement recouverte d'une couche de passivation, non représentée. Les anneaux de de garde 52, comme cela est représenté, peuvent s'étendre en profondeur à l'aide de plages métalliques empilées dans tous les niveaux de métal de la technologie, sept en CMOS 65 nm, interconnectées par des vias. Les vias peuvent être agencés autour de chaque pixel, selon un pas apte à parfaire la fonction d'écrantage.

L'épaisseur du substrat 60 peut être choisie pour limiter les réflexions internes des ondes THz sur la face arrière du substrat, notamment des ondes produites par les émetteurs qui pourraient perturber les récepteurs voisins. Cet objectif est atteint avec une épaisseur au plus égale à la moitié de la longueur d'onde dans le silicium, à savoir 0,15 mm.

Les anneaux de garde et le choix d'une épaisseur spécifique du substrat permettent d'éviter, dans le domaine THz, les problèmes de perturbation parasites pouvant provenir de l'intégration d'un émetteur dans une matrice de récepteurs.

Comme on l'a représenté pour une paroi de l'un des anneaux de garde, les plages métalliques peuvent être structurées pour former une cavité 62. La cavité 62 peut loger des pistes conductrices et des composants électroniques servant à l'exploitation des pixels. En effet, la largeur de deux anneaux de garde juxtaposés est de l'ordre de 60 µm, ce qui, en une technologie de 65 nm, offre suffisamment de place pour loger la majorité des conducteurs et composants électroniques requis localement pour exploiter les pixels. Cette configuration permet de réduire au strict minimum les conducteurs métalliques dans la zone vide autour des antennes, qui perturberaient les propriétés optiques.

## Revendications

1. Capteur pour système d'imagerie térahertz, intégralement réalisable dans une technologie de semi-conducteurs et comprenant :
une zone d'analyse (12) ;
un substrat plan (60) en matériau semi-conducteur transparent aux rayonnements térahertz, disposé à une distance de la zone d'analyse (12) sans interposition de lentille entre le substrat et la zone d'analyse ;
une pluralité de récepteurs d'un rayonnement térahertz agencés selon une matrice (10) dans le substrat ;
au moins une source ponctuelle cohérente de rayonnement térahertz agencée dans le substrat, la distance entre le substrat et la zone d'analyse étant telle que le rayonnement émis par la source selon un cône d'émission (C) soit réfléchi par la zone d'analyse (12) sur la matrice de récepteurs selon un disque qui est un hologramme ; et
des moyens pour opérer une transformée de Fourier inverse de l'hologramme reçu par la matrice de récepteurs.

2. Capteur selon la revendication 1, dans lequel le pas de la matrice est au moins égal à la moitié de la longueur d'onde du rayonnement à l'intérieur du substrat, et la source prend la place d'un récepteur dans la matrice.

3. Capteur selon la revendication 1, dans lequel les récepteurs sont des récepteurs hétérodynes synchronisés sur un même oscillateur local, d'où il résulte que chaque récepteur fournit une information de phase représentative de la distance parcourue depuis la source par le rayonnement reçu.

4. Capteur selon la revendication 2, dans lequel les récepteurs et la source ont une configuration hexagonale et sont agencés selon une matrice en nid d'abeille.

5. Capteur selon la revendication 2, comprenant plusieurs sources régulièrement espacées dans la matrice de sorte que chaque source soit séparée d'une source voisine la plus proche par plusieurs récepteurs.

6. Capteur selon la revendication 2, comprenant un support tubulaire (16) configuré pour être appuyé sur un objet à analyser et maintenir le capteur à une distance fixe de l' obj et.

7. Capteur selon la revendication 2, dans lequel le substrat a une épaisseur au plus égale à la moitié de la longueur d'onde du rayonnement térahertz à l'intérieur du substrat, et comprend :
une face active incluant des niveaux de métal ;
une face arrière configurée pour être orientée vers la zone d'analyse ;
et chaque récepteur et source comprend :
une antenne annulaire (50) formée dans un niveau de métal de la face active, la circonférence moyenne de l'antenne étant au moins égale à la moitié de la longueur d'onde du rayonnement térahertz à l'intérieur du substrat ; et
un anneau de garde (52) entourant l'antenne à la périphérie du récepteur ou source, formé à partir de plages métalliques empilées sur plusieurs niveaux de métal.

8. Capteur selon la revendication 7, dans lequel l'anneau de garde (52) comprend des plages métalliques structurées pour former une cavité (62) abritant des pistes conductrices et des composants électroniques servant à exploiter les récepteurs et la source.

9. Procédé d'imagerie térahertz à réflexion sans lentille utilisant un capteur selon la revendication 5, comprenant les étapes suivantes :
activer les sources une par une selon une séquence résultant en l'illumination d'une même zone de la zone d'analyse selon des angles différents ;
collecter les images résultantes reçues par le capteur dans le domaine fréquentiel ; et
traiter les images résultantes du domaine fréquentiel pour produire une image améliorée dans le domaine spatial.

10. Procédé selon la revendication 9, dans lequel les images résultantes sont traitées par une technique de ptychographie utilisant seulement des informations d'amplitude fournies par les récepteurs.

11. Procédé selon la revendication 9, dans lequel les images résultantes sont traitées par une technique de microscopie à synthèse d'ouverture utilisant des informations d'amplitude et de phase fournies par les récepteurs.

## Patentansprüche

1. Sensor für ein Tetrahertz-Bildgebungssystem, das in einer Halbleitertechnologie vollständig realisierbar ist und umfassend:
einen Analysebereich (12);
ein ebenes Substrat (60) aus einem Halbleitermaterial, das für Tetrahertzstrahlung durchlässig ist, das in einem Abstand von dem Analysebereich (12) angeordnet ist, ohne Einschalten einer Linse zwischen dem Substrat und dem Analysebereich;
eine Vielzahl von Empfängern einer Tetrahertzstrahlung, die entlang einer Matrix (10) in dem Substrat angeordnet sind;
mindestens eine mit der Tetrahertzstrahlung kohärente Punktquelle, die in dem Substrat angeordnet ist, wobei der Abstand zwischen dem Substrat und dem Analysebereich derart ist, dass die durch die Quelle emittierte Strahlung entlang einem Sendekegel (C) durch den Analysebereich (12) auf die Matrix der Empfänger entlang einer Scheibe, die ein Hologramm ist, reflektiert wird; und
Mittel zum Durchführen einer inversen Fouriertransformation des Hologramms, das von der Matrix der Empfänger empfangen wurde.

2. Sensor nach Anspruch 1, wobei der Abstand der Matrix mindestens gleich der Hälfte der Wellenlänge der Strahlung im Inneren des Substrats ist, und wobei die Quelle den Platz eines Empfängers in der Matrix einnimmt.

3. Sensor nach Anspruch 1, wobei die Empfänger Schwebungsempfänger sind, die auf einem gleichen lokalen Oszillator synchronisiert sind, wo jeder Empfänger eine Phaseninformation bereitstellt, die den zurückgelegten Abstand von der Quelle durch die empfangene Strahlung darstellt.

4. Sensor nach Anspruch 2, wobei die Empfänger und die Quelle eine hexagonale Konfiguration aufweisen und entlang einer Matrix in Wabenform angeordnet sind.

5. Sensor nach Anspruch 2, umfassend mehrere Quellen, die gleichmäßig in der Matrix derart beabstandet sind, sodass jede Quelle von einer benachbarten nächstgelegenen Quelle durch mehrere Empfänger getrennt ist.

6. Sensor nach Anspruch 2, umfassend einen röhrenförmigen Träger (16), der konfiguriert ist, um auf ein zu analysierendes Objekt aufgestützt zu werden und den Sensor in einem festen Abstand von dem Objekt zu halten.

7. Sensor nach Anspruch 2, wobei das Substrat eine Dicke von höchstens gleich der Hälfte der Wellenlänge der Tetrahertzstrahlung im Inneren des Substrats aufweist, und umfasst:
eine aktive Seite, die eine Metallebene enthält;
eine Rückseite, die konfiguriert ist, um in Richtung des Analysebereichs ausgerichtet zu sein;
und wobei jeder Empfänger und jede Quelle umfassen:
eine ringförmige Antenne (50), die in einer Metallebene der aktiven Seite ausgebildet ist, wobei der mittlere Umfang der Antenne mindestens gleich der Hälfte der Wellenlänge der Tetrahertzstrahlung im Inneren des Substrats beträgt; und
einen Schutzring (52), der die Antenne im Umkreis des Empfängers oder der Quelle umgibt, der aus Metallflächen gebildet ist, die auf mehreren Metallebenen gestapelt sind.

8. Sensor nach Anspruch 7, wobei der Schutzring (52) die Metallflächen umfasst, die strukturiert sind, um einen Hohlraum (62) zu bilden, der die Leiterbahnen und die elektronischen Komponenten beherbergt, die zum Betreiben der Empfänger und der Quelle dienen.

9. Verfahren der Tetrahertz-Bildgebung der Reflexion ohne Linse, unter Verwendung eines Sensors nach Anspruch 5, umfassend die folgenden Schritte:
Aktivieren der Quellen, eine nach der anderen, gemäß einer Sequenz, die zu einer Beleuchtung eines gleichen Bereichs vom Analysebereich entlang unterschiedlicher Winkel führt;
Sammeln der resultierenden Bilder, die durch den Sensor in der Frequenzdomäne empfangen werden; und
Verarbeiten der resultierenden Bilder von der Frequenzdomäne, um ein verbessertes Bild in der räumlichen Domäne herzustellen.

10. Verfahren nach Anspruch 9, wobei die resultierenden Bilder durch eine Ptychographie-Technik verarbeitet werden, unter Verwendung nur der Amplitudeninformationen, die durch die Empfänger bereitgestellt werden.

11. Verfahren nach Anspruch 9, wobei die resultierenden Bilder durch eine Mikroskopie-Technik mit synthetischer Apertur verarbeitet werden, unter Verwendung der Amplituden- und Phaseninformationen, die durch die Empfänger bereitgestellt werden.

## Claims

1. A sensor for a terahertz imaging system integrally achievable in semiconductor technology, comprising:
an analysis area (12);
a flat substrate (60) of semiconductor material located at a distance from the analysis area, with no lens interposed between the substrate and the analysis area (12);
a plurality of terahertz radiation receivers arranged in an array (10) on the substrate;
at least one coherent spot source of terahertz radiation arranged on the substrate, wherein the distance between the substrate and the analysis area is such that the radiation emitted by the source according to a reflection cone is reflected by the analysis area (12) towards the array of receivers according to a disc forming a hologram; and
means for operating an inverse Fourier transform of the hologram measured by the array of receivers.

2. The sensor according to claim 1, wherein the pitch of the array is at least half the wavelength of the radiation within the substrate, and the source takes the place of a receiver in the array.

3. The sensor according to claim 1, wherein the receivers are heterodyne receivers synchronized on the same local oscillator, whereby each receiver provides phase information representative of the distance travelled by the received radiation from the source.

4. The sensor according to claim 2, wherein the receivers and the source have a hexagonal configuration and are arranged in a honeycomb matrix.

5. The sensor according to claim 2, comprising a plurality of sources evenly distributed in the array such that each source is separated from a closest neighboring source by multiple receivers.

6. The sensor according to claim 2, comprising a tubular support (16) configured to be applied to an object to be analyzed and to maintain the sensor at a fixed distance from the object.

7. The sensor according to claim 2, wherein the substrate has a thickness at most equal to half the wavelength of the terahertz radiation within the substrate, and comprises:
an active face including metal levels;
a back face configured to be oriented towards the analysis area;
and each of the receivers and source includes:
an annular antenna (50) formed in a metal level of the active face, the average circumference of the antenna being at least half the wavelength of the terahertz radiation within the substrate; and
a guard ring (52) surrounding the antenna at the periphery of the receiver or source, formed from metal patterns stacked in several levels of metal.

8. The sensor according to claim 7, wherein the guard ring (52) comprises metal patterns structured to form a cavity (62) housing conductor tracks and electronic components for controlling the receivers and the source.

9. A method of lensless reflection terahertz imaging using a sensor according to claim 5, comprising the following steps:
actuating the sources one at a time in a sequence resulting in the illumination of a same area of the analysis area from different angles;
collecting the resulting images measured by the sensor in the frequency domain; and
processing the resulting frequency domain images to produce an enhanced space domain image.

10. The method according to claim 9, wherein the resulting images are processed by a ptychography technique using only amplitude information provided by the receivers.

11. The method according to claim 9, wherein the resulting images are processed by a synthetic aperture microscopy technique using amplitude and phase information provided by the receivers.
